# EUROPEAN PATENT APPLICATION

(11) **EP 0 648 496 A1**
(43) Date of publication of application: **19.04.1995**
(21) Application number: 94202902.6
(22) Date of filing: 07.10.1994
(51) Int. Cl.: A61K 35/78

(54) **Therapeutic compositions for use in the treatment of skin lesions and method of making same**

(30) Priority: 15.10.1993 GB 9321341
(71) Applicant: Bristol-Myers Squibb Company, New York, N.Y. 10154 (US)
(72) Inventor: Patrick, Kevin, Upton, Wirral Merseyside L49 4QX (GB); Jacques, Elizabeth Joan, Chester CH2 3LQ (GB)
(74) Representative: Mays, Julie

(57) **Abstract**

The present invention relates to therapeutic compositions for use in the treatment of skin lesions and in particular dermal ulcers, wounds and burns. A second aspect relates to the preparation of said compositions and a further aspect relates to the use of medicinal herbs in the treatment of skin lesions. The invention provides a composition comprising from 0.1 to 5 % by weight of a flavonoid glycoside or free sugar thereof and from 1 to 5 % by weight of a gelling agent.

## Description

A first aspect of the present invention relates to therapeutic compositions for use in the treatment of skin lesions and in particular dermal ulcers, wounds and burns. A second aspect relates to the preparation of said compositions and a further aspect relates to the use of medicinal herbs in the treatment of skin lesions.

There are many known medicinal products based on plant extracts. These products tend to be simple aqueous alcohol or oil based extractions from the plants themselves. A problem with the treatment of skin lesions with such products is that it is difficult and inconvenient to maintain contact between the extract and the wound due to the product form. In addition known treatments are extractions from large numbers of plants which may not all be therapeutic to chronic wounds such as dermal ulcers. An additional disadvantage is that aqueous extracts are liable to contamination by bacteria and thus have a very short useful life.

In RO 93177 there is disclosed a solution for treatment of leg ulcers consisting of an aqueous alcohol extract obtained from *Calendula officinalis L*., root of *Symphytum officinale L*. and stem with root of Echinacea purpurea. The solution may be applied by poultice. The disadvantages of this type of treatment are that the sterility of the solution cannot be guaranteed due to potential bacterial contamination during use and treatment of wounds by poultice is inconvenient for both patient and carer due to non-adherence of the poultice to the wound.

We have now found that skin lesions can be therapeutically treated using compositions comprising flavanoid glycosides or free sugars of flavonoid glycosides and gelling agents.

Accordingly the invention provides a therapeutic composition for use in the topical treatment of skin lesions comprising: from 0.1 to 5 % by weight of flavonoid glycosides or free sugars thereof
and
from 1 to 5 % by weight of a gelling agent.

In particular the invention provides a therapeutic composition in gel-form for use in the topical treatment of skin lesions comprising:
from 0.1 to 5 % by weight of flavonoid glycosides or free sugars thereof
and
from 2 to 10 % by weight of a gelling agent.

In a preferred embodiment the flavonoid glycoside is for example a glycoside of a flavonol or a flavone for example a glycoside of quercetin as found in extracts of *Calendula officinalis L*. Accordingly the invention provides a composition for the treatment of skin lesions comprising an aqueous extract of *Calendula officinalis L*. Preferably the composition comprises from 0.1% to 50% by weight of a freeze dried aqueous extract of *Calendula officinalis*. The free plant sugar is for example rutinose as found in botanical sources such as Buckwheat (*Fagopyrum esculentum*) or the French bean (*Phaseolus vulgaris*). Alternatively rutinose may be prepared from other commercial synthetic sources.

Flavonoid glycosides suitable for use in the present invention may be represented by the following strucural formula: 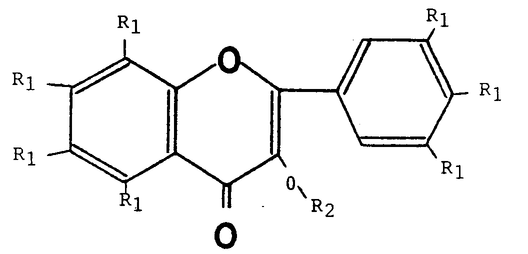
where R₁ is H or OH and
R₂ is a sugar group
A preferred flavonoid glycoside is rutin which may be represented by the following structural formula: 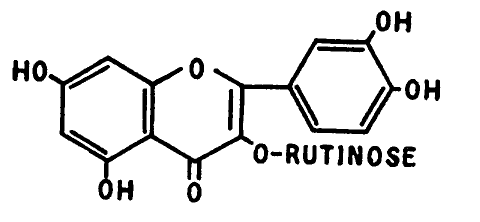
rutin (buckwheat)
In rutin the sugar group R₂ is rutinose (6-0-λ-L-rhammosyl-D-glucose) which may be represented by the following structural formula:
Rutinose: 6-O-λ-L-rhammosyl-D glucose (glycosides) 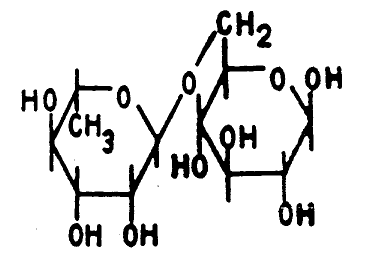
Other examples of glycosides of quercetin suitable for use in compositions of the present invention are as follows:
3 - Arabinoside
3 - Xyloside
3 - Glucoside also known as isoquercitrin
3 - Galactoside
3 - Rhamnoside also known as quercitrin
3 - Glucuronide
3 - Rutinoside also known as rutin
7 - Glucoside
4'- Glucoside
Examples of free plant sugars derived from flavonoid glycosides suitable for use in the present invention are glucose and fructose and the disaccharide sucrose together with traces of xylose, rhamnose and galactose. Others are the uronic acids, glucuronic and galacturonic acids and mannose. Free sugars are also commonly found as components of glycosides, for example these include glucose, galactose, xylose, arabinose and rhamnose. The flavonoid glycosides and/or free sugars thereof are preferably present in the composition at a level of from 0.1% to 5% by weight.

Gelling agents suitable for use in compositions according to the present invention are:
Cellulose derivatives for example carboxymethylcellulose, sodium blanose, hydroxypropylmethylcellulose (hypromellose), methyl cellulose (Methocel), and hydroxyethylcellulose (Natrosol);
Pectin;
Hyaluronic acid and salts thereof;
Alginates;
Fumed Silica (aerosil);
Polyacrylic acid (Carbopol) and salts thereof and
other ionic and nonionic gels. The gelling agent is preferably present in the compositions at a level from 1% to 10%, preferably 2% to 10% by weight.

Other excipients for the purposes of providing humectancy and/or preservation and stabilisation of the gel may be included. Suitable humectants include glycerol, propylene glycol and sorbitol. Stabilising additives include sodium metabisulphate, EDTA and ascorbic acid. Suitable antimicrobial preservatives may be included to enable multiple use of the gel. Typical preservatives include parahydroxybenzene and esters, phenoxyethanol, bromopol, imidazolidinyl urea, benzoic acid (and salts), benzalkonium chloride, chlorhexidinegluconate and ethanol.

As the compositions according to the invention are gelled they can be applied directly to the wound for example in combination with an occlusive or semi-occlusive dressing. EP 92 999 B (E.R. Squibb and Sons), EP 130 061 B (E.R. Squibb and Sons), EP 190 814 B (E.R. Squibb and Sons) and EP 302 536 B (E.R. Squibb and Sons) incorporated herein by reference describe occlusive dressings for use in wound healing. The compositions according to the invention could be combined with the compositions described therein. The sugars of flavonoid glycosides may thus be in prolonged intimate contact with the wound allowing constant exposure to the therapeutic material. This gives the additional advantage that dosage of the active ingredients can be controlled. Gel formulations also have a reduced risk of bacterial contamination because the osmotic potential of the gel medium provides a reduced activity of water (A_{w}) and thus an unfavourable environment for bacterial growth.

Alternative product forms are creams, ointments, water soluble films, sprays, foams, aerosols, pastes, film forming solutions and emulsions. In an adhesive or non-adhesive dressing the composition may be integrally mixed with the base material or coated onto the surface of the base material.

A preferred source of flavanoid glycosides or free sugars of flavonoid glycosides is an aqueous infusion of *Calendula officinalis L*. This can be prepared by placing the flower heads in cold water for a prolonged period for example 100g of dried flower heads in 1 litre of distilled water for 12 hours. The resulting mash can be filtered to yield an extract of a clear dark brown fluid with a sweet herbal smell. To prevent bacterial contamination and to preserve the extract it can be freeze dried for example in an SP4 Freeze Drier ex Chemical Labs Instruments Ltd., Ilford, Sussex. The freeze dried extract can then be sealed under nitrogen in amber glass.

A second aspect of the invention relates to the preparation of a composition for the topical treatment of skin lesions, said process comprising the steps of:
(i) preparing an aqueous infusion of *Calendula Officinalis L.;*
(ii) freeze drying said infusion;
(iii) rehydrating the freeze dried infusion with mixing; and
(iv) adding a gelling agent to the rehydrated infusion.

A third aspect of the invention relates to the use of a composition comprising a sugar of flavonoid glycoside and a gelling agent in the preparation of a medicament for use in the treatment of skin lesions.

The invention will now be illustrated by the following non-limiting examples.

### Example 1

The following example shows the therapeutic effect of flavonoid glycosides and free sugars of flavonoid glycosides on skin lesions. An indication of the rate of wound healing is given by the perfusion level associated with the wound as measured by laser Doppler Flowmetry (LDF). Capillary growth in wound healing is initiated by growth from pre-existing blood vessels. Blood vessel development stems from the granulation tissue of newly healing wounds progressing to full neovascularisation. The appearance of viable blood vessels allows blood flow and subsequent perfusion of the surrounding tissues. Wound healing progresses by provision of an efficient blood supply providing the necessary metabolites of tissue repair. LDF uses low power monochromatic laser light and allows non-invasive reproducible measurement of blood flow in the wound without affecting healing. The following results were obtained by using a Perimed Periflux PF3 laser Doppler perfusion monitor. The monitor was used to measure perfusion levels in a porcine acute cutaneous full thickness wound model, over a period of two to fourteen days. In the study six wounds (0.5 cm in diameter and extending down to the level of the muscle fascia) were inflicted on each of the pigs in the study. A gel formulation, formulation A, according to the invention was prepared as follows. Freeze dried *Calendula officinalis L*. was added to sterile pyrogen free water in a sterile glass container and mixed with a propeller mixer. Hyrdroxyethyl cellulose was then slowly added with mixing to form a gel. The resulting gel was mixed for a further 10 minutes.

| | |
|---|---|
| Formulation A (by weight) | 96% sterile pyrogen free water |
| | 3% Hydroxyethyl cellulose |
| | 1% *Calendula officinalis L*. |

A second gel formulation, formulation B, according to the invention was prepared as follows. Rutinose in the form of rutinose hepataacetate was added to sterile pyrogen free water in a sterile glass container and warmed for 15 minutes until completely dissolved. Rutinose , such as in the form of rutinose heptaacetate, is prepared from the enzymatic hydrolysis of rutin using rhamnodiastase. Rutin may be obtained from botanical sources such as Buckwheat (*Fagopyrum esculentum*) or the French bean (*Phaseolus vulgaris)*. Alternatively rutinose may be prepared from commercial synthetic sources. The resulting solution was filtered to remove surface scum followed by a sterile filtration using a 0.2 micron millipore filter. A vortex was then created in the rutinose solution with a propeller mixer and hydroxyethyl cellulose slowly added to form a gel. The gel was then mixed for a further 10 minutes.

| | |
|---|---|
| Formulation B (by weight) | 96% sterile pyrogen free water |
| | 3% hydroxyethyl cellulose |
| | 1% rutinose (sugar group from rutin (flavonoid glycoside) as heptaacetate) |

A control formulation, Formulation C was prepared in the above manner.

| | |
|---|---|
| Formulation C (by weight) | 97% sterile pyrogen free water |
| | 3% hydroxyethyl cellulose |

The hydroxyethyl cellulose gel formulation was chosen as a neutral vehicle to deliver the active ingredients into the wound. The gel was held in place by a placebo film dressing consisting of a polyurethane film coated with a polyisobutylene adhesive. (Vistanex dressing V5)

The measured perfusion levels are shown in Table I. These results show a statistically significant increase in perfusion levels of *Calendula officinalis L*. treated wounds and rutinose treated wounds over the control in a porcine acute cutaneous full thickness wound model. The increase in perfusion level appears at day 8 increasing to a peak at day 11. This type of increased perfusion is known to enhance the healing capabilities of wounds.

### Example 2

The percentage of re-epithelialization of the wounds of example 1 was measured in control porcine acute cutaneous full thickness wound model. The results are shown in Table 2. The results show a statistically higher rate of reepithelialization in the porcine full thickness wound model control pigs for wounds treated with rutinose, and a biologically higher rate of re-epithelialization with *Calendula officinalis L*. treated wounds.

### Example 3

The following example shows the therapeutic effect of a free sugar of a flavonoid glycosides and *Calendula officinalis L* on skin lesions. Example 1 was repeated with formulations A and C being applied to porcine full thickness wounds. The wounds were biopsied at days 4, 8, 11 and 14 for histological assessment of the wound healing process.

### Histological Results

On day 8, all samples irrespective of wound treatment displayed the presence of an acute and superficial inflammatory infiltrate with superficial eschar. Differences were noted in the numbers of blood vessels present and the percentage of fibrous granulation tissue occupying the wound matrix overall. Peak perfusion levels were seen in the wound tissue from treatment with formulation A according to the invention.

The percentage of reepithelialisation in the wounds was determined by quantitative measurement of the newly forming epidermis over the wound matrix. This was determined using an eyepiece graticule (4 sections/per treatment/per time point). The results are shown in the table below.

### Reepithelialisation results

| **Treatment Day** | **Formulation A %** | **Formulation C %** |
|---|---|---|
| 4 | 12.5 | 15.40 |
| 4 | 13.3 | 16.70 |
| 4 | 12.5 | could not calculate |
| 4 | 8.0 | could not calculate |
| 8 | 43.75 | 53.30 |
| 8 | 56.25 | 66.70 |
| 8 | 41.20 | 50.00 |
| 8 | 41.20 | 50.00 |
| 11 | 100.00 | 85.71 |
| 11 | 100.00 | 81.91 |
| 11 | could not calculate | 100.00 |
| 11 | could not calculate | 100.00 |
| 14 | 100.00 | 100.00 |
| 14 | 100.00 | 100.00 |
| 14 | 100.00 | 100.00 |
| 14 | 100.00 | 100.00 |

These results show that complete reepithelialisation was achieved by Formulation A according to the invention by day 8 compared to day 11 with the control.

### Example 4

A thin-layer chromatograph of a 3% freeze dried *Calendula officinalis* solution was prepared by running in a solvent of butanol: acetic acid: water in the ratio, 4:1:1. The chromatograph showed that a mixture of flavonoid glycosides, aglycones and free sugars was present in the solution.

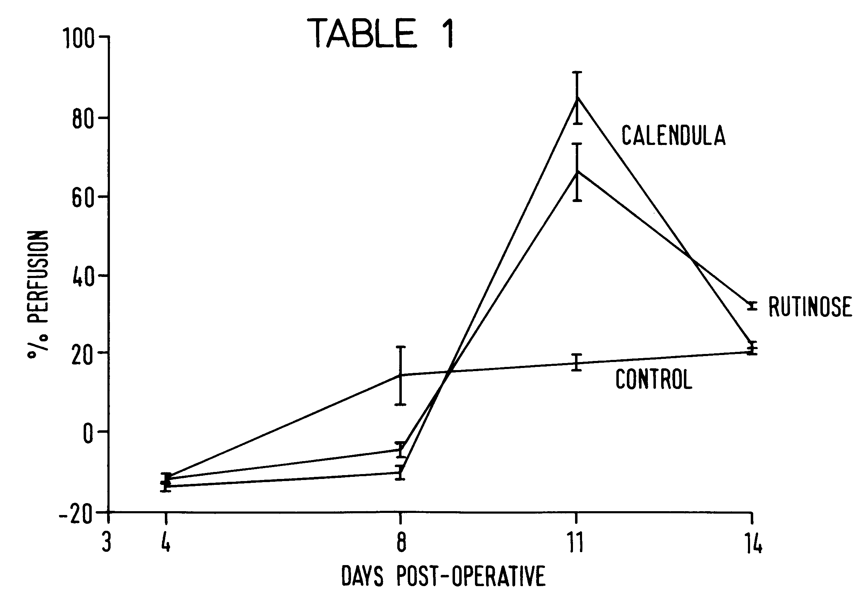

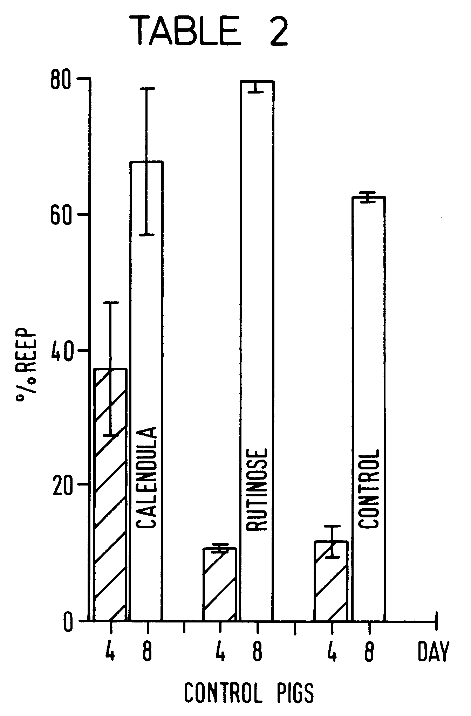

## Claims

1. A composition comprising:
from 0.1 to 5 % by weight of a flavonoid glycoside or a free sugar thereof and
from 1 to 5 % by weight of a gelling agent.

2. A composition in gel-form comprising:
from 0.1 to 5 % by weight of a flavonoid glycoside or a free sugar thereof and
from 2 to 10 % by weight of a gelling agent.

3. A composition as claimed in claim 1 or claim 2 wherein the flavonoid glycoside is rutin.

4. A composition as claimed in claim 1 or claim 2 wherein the free sugar is rutinose.

5. A composition for the treatment of skin lesions comprising a freeze dried aqueous extract of *Calendula officinalis L*.

6. A composition as claimed in claim 5 wherein the composition comprises from 0.1 to 50% by weight of a freeze dried aqueous extract of *Calendula officinalis L* and further comprises from 1 to 10% by weight of a gelling agent.

7. The preparation of a composition for the topical treatment of skin lesions, said process comprising the steps of:
(i) preparing an aqueous infusion of *Calendula Officinalis L.;*
(ii) freeze drying said infusion;
(iii) rehydrating the freeze dried infusion with mixing; and
(iv) adding a gelling agent to the rehydrated infusion.

8. Use of a composition comprising a flavonoid glycoside or a free sugar of a flavonoid glycoside and a gelling agent in the preparation of a medicament for use in the prevention and treatment of skin lesions.

9. Use of a composition comprising an aqueous extract of *Calendula officinalis L*. and a gelling agent in the preparation of a medicament for use in the prevention and treatment of skin lesions.

10. Use of a composition as claimed in claim 8 wherein the flavonoid glycoside is rutin.
